# EUROPEAN PATENT SPECIFICATION

(11) **EP 1 123 711 B1**
(45) Date of publication and mention of the grant of the patent: **19.11.2008**
(21) Application number: 99949324.0
(22) Date of filing: 20.10.1999
(51) Int. Cl.: A61K 39/39, A61K 39/145, A61K 39/15, A61K 39/165, A61K 39/20, A61K 39/21, A61K 39/10, A61K 39/07, A61K 39/02, A61K 39/108, A61K 39/118, A61K 39/00, A61K 39/015, A61K 39/012, A61K 39/116, A61K 39/295, A61P 31/04, A61P 31/12, A61P 31/00

(54) **Attenuated toxins as adjuvants**
Attenuierte Toxine als Adjuvantien
Toxines atténuées comme un adjuvant

(30) Priority: 21.10.1998 JP 30021998
(43) Date of publication of application: 16.08.2001
(73) Proprietor: THE KITASATO INSTITUTE, Tokyo 108-8642 (JP)
(72) Inventor: AIZAWA, Chikara, Research Center for Biologicals, Kitamoto-shi, Saitama 364-0026 (JP); SUZUKI, Yuziro, Research Center for Biologicals, Kitamoto-shi, Saitama 364-0026 (JP); SATO, Taka-aki, Research Center for Biologicals, Kitamoto-shi, Saitama 364-0026 (JP); WATANABE, Koji, Research Center for Biologicals, Kitamoto-shi, Saitama 364-0026 (JP); HATTORI, Nobuaki, Research Center for Biologicals, Kitamoto-shi, Saitama 364-0026 (JP); TANAKA, Yoshitake, Research Center for Biologicals, Kitamoto-shi, Saitama 364-0026 (JP); OMURA, Satoshi, The Kitasato Institute, Tokyo 108-8642 (JP)
(74) Representative: Grünecker, Kinkeldey, Stockmair & Schwanhäusser Anwaltssozietät
(86) International application number: PCT/JP1999/005789
(87) International publication number: WO 2000/023107

(56) References cited:
- WO-A-96/34623
- WO-A1-95/17211
- WO-A1-98/42375
- WO-A2-95/34323
- LIANG X. ET AL.: 'Cholera Toxin as a Mucosal Adjuvant: Glutaraldehyde Treatment Dissociates Adjuvanticity from Toxicity' J. IMMUNOL., vol. 143, no. 2, 1989, pages 484 - 490, XP002921957
- DE GRAVE D. ET AL.: 'Enhancement of the Humoral Immunity Against HBSAG by Pertussis Toxin' MED. FAC. LANDBOUWW. RIJKSUNIV. GENT, vol. 54, no. 4B, 1989, pages 1553 - 1555, XP000603588
- RYAN M. ET AL.: 'Pertussis Toxin Potentiates Th1 and Th2 Responses to Co-injected Antigen: Adjuvant Action is Associated with Enhanced Regulatory Cytokine Production and Expression of the Co-stimulatory Molecules B7-1, B7-2, and CD28' INT. IMMUNOL., vol. 10, no. 4, May 1998, pages 651 - 662, XP002921958
- Mc Clurg WM et al., Formaldehyde replaces glutaraldehyde in porcine bioprosthetic heart valves. J Heart Valve Dis. 1996, 5: 343
- Holla BS et al., Sutides on arylfuran derivatives. 1998, 53:531.
- Shiurba RA et al., Immunocytochemistry of formalin-fixed human brain tissues: microwave irradiation of free-floating sections. 1998, 2:109.
- SATO Y ET, FUKUMI H AND KIMURA M: "Development of a pertussis component vaccine in Japan" THE LANCET, vol. 1, 1984, pages 122-126, XP009098437

## Description

### Technical Field

The present invention relates to vaccine preparations that contain an attenuated toxin as the adjuvant and that are useful for treating and preventing diseases.

### Background Art

Vaccines have been used to prevent various diseases, and tremendous results have been obtained. Nonetheless, vaccines also have side effects and there are many cases in which vaccines are not very effective. Thus, there is a strong need for improved vaccines.

Currently, many types of vaccines, for human or other animals, use pathogens or parts thereof as antigenic materials for vaccines. Thus, there is no denying the possibility that vaccines may be contaminated with constituents of pathogens or ingredients of the growth medium for the pathogens. These contaminants can provoke undesirable side reactions in vaccination. In addition, antigenic domains themselves, pertaining to immunity, can induce side reactions when inoculated in large quantity.

In an effort to avoid such side reactions as much as possible and manufacture vaccines excellent in safety, those in the art have reduced the inoculum size of vaccine antigen, improved the purity of antigen for vaccination, and/or administered a vaccine by a route other than injection.

For example, WO96/34623 discloses acellular pertussis vaccines comprising pertussis toxin, filamentous haemagglutinin, pertactin, and fimbrial agglutinogens".

However, generally such revision results in the reduction of immunogenic activity of the vaccine. Previously, adjuvants have been used as an effective countermeasure to this problem. However, there still remain some problems to be solved, such as improving the effectiveness and safety of the adjuvant.

So far most vaccine inoculations are given by injection. This results in elevation of antibody titer in the blood. When the titer is maintained at a high level, it inhibits the propagation of pathogenic microorganisms and thereby preventing diseases associated with the organisms. On the other hand, various viruses, such as influenza virus, as well as bacteria infect via mucous membranes of the respiratory tract. To prevent such diseases at an early stage of infection, vaccines capable of significantly enhancing local immunity, in the mucous membrane rather than in the blood, are preferred. To achieve this goal, excellent adjuvants that help potentiate local immunity are desired. In other words, the development of an excellent adjuvant, that is effective and safe and that matches the type of antigen and vaccination route to be used, is an important subject in the development of new and improved vaccines.

Noteworthy inoculation routes other than injection include oral, percutaneous and intranasal administration. Injections must be conducted by medical staffs. Therefore, the vaccine inoculation by injection becomes problematic, for example, when it is necessary to vaccinate many people under a condition with poor medical facilities. On the other hand, oral, percutaneous or intranasal administration can be performed with the instruction of a medical professional but without direct assistance as far as vaccine preparations are available. However, in general, sufficient immunological stimulation is hardly attainable with these routes of administration, and therefore suitable adjuvants for such routes are demanded.

Previously, aluminum compounds (aluminum sulfate, aluminum hydroxide, aluminum phosphate, etc.) have been widely used as adjuvants for vaccines. Currently, the gel of aluminum compound is almost the only adjuvant available for vaccines of human use. However, there are some problems in regard to the aluminum adjuvant, and thus improvements are needed. Illustrative problems are pointed out as followings:
1) Problems associated with the production of aluminum adjuvant as well as its handling are as follows: since the quality of aluminum adjuvant tends to vary from one production lot to another, it is not suited to large scale manufacturing. Moreover, the compound tends to be interfering in column chromatography of co-existing proteins, and is therefore inconvenient to handle.
2) Another problem associated with the aluminum adjuvant is that while it excels in potentiating the humoral immunity, the compound is less effective for potentiating cellular immunity. Thus the types of antigens to be used together are limited.

Research and development of new types of adjuvants, such as bacterial toxins, that overcome the drawbacks discussed above are in progress. Some illustrative examples are as follows:
1. Bacterial toxins, such as cholera toxin, and the like;
2. Substances having detergent activity, such as saponins, higher fatty acids, and the like;
3. Constituents of microorganisms or plants, such as BCG, muramyl peptide, and the like;
4. Functional proteins including cytokines, such as interleukin, heat shock protein, and the like;
5. Synthetic polyanion, polycation, and the like; and
6. Micro-carriers and the like;.

It is well known that certain bacterial toxins have adjuvant activity, namely, activity of enhancing production of antigen-specific antibody. Among them, cholera toxin exhibits relatively high adjuvant activity (J. Holmgren et al., Vaccine 11, 1179-1184, 1993). The present inventors have been studying and developing vaccines containing adjuvants such as cholera toxin, *E*. *coli* heat-labile toxin, and the subunits thereof (Unexamined Published Japanese Patent Application (JP-A) No. Hei 2-243633).

In addition to these studies, the ongoing research and development, which relates to vaccines containing cholera toxin, *E*. *coli* heat-labile toxin, pertussis toxin, and such as the adjuvant, includes the following examples: influenza vaccine (K. Komase et al., Vaccine 16, 248-254, 1998; A. S. Tamura et al., Eur. J. Immunol. 21, 1337-1344, 1991), herpes simplex virus vaccine (C. M. Richards et al., Vaccine 15, 1065-1069, 1997), HIV-I vaccine (I. M. Belyakov et al. Proc. Natl. Acad. Sci. USA 95, 1709-1714, 1998), *Helicobacter pylori* vaccine (R. Weltzin et al., Vaccine 15, 370-378, 1997), toxoplasma vaccine (I. Bourgiun et al., FEMS Immunol. Med. Microbiol 12, 121-126, 1995), measles vaccine (C. D. Partidos et al., Imunology 89, 483-487, 1996), poliovirus vaccine, etc.

However, the usage of vaccines added with bacterial toxins as adjuvant in clinical practice might be of problem, because bacterial toxins are originally toxic to humans. The present inventors assumed that the toxins can be used safely for vaccines because the effective concentration of adjuvant toxins is sufficiently below the level where the toxins exhibit toxicity to humans and other animals. Thus, the present inventors studied the possible problems associated with repeated administration of the adjuvanted intranasal vaccine and reported the result previously (S. Tamura et al., Vaccine 15, 1784-1790, 1997). Nonetheless, many experts have expressed concerns about safety of toxin-adjuvant vaccine due to, for example, the difference of susceptibility to cholera toxin between human and experimental animals as well as the possibility of causing diarrhea (M.M. Levine et al. Microb. Rev. 47, 510-550, 1983).

The following methods have been proposed in order to take advantage of excellent activity of bacterial toxin in enhancing immunity and simultaneously to solve the problem of safety. However, none of the methods come close to reaching the goal of realizing both safety and adjuvant activity.

### (1) Attenuation by chemical or physical treatment:

A method to reduce the activity of toxin without losing the adjuvant activity - wherein the toxin is treated with formalin, glutaraldehyde, acid, alkali, etc. or treated at a temperature severe for the toxin. For example, "attenuation by formalin treatment is known as described in De Grave, D., et al., Med. Fac. Landbouww. Rijksuniv. Gent, Vol. 54, No. 4b, 1989, pp. 1553-1555". It is known that cholera toxin treated with glutaraldehyde has a toxic activity reduced to about 1/1000 as compared with the natural one while maintaining adjuvant activity (X. Liang et al., J. Immunol. 143, 484-490, 1989). However, toxin activity reduced to 1/1000 relative to that of the natural toxin is evaluated not to be sufficiently safe.

### (2) Search for cross-reacting mutant proteins produced by mutant strains:

A method for searching cross-reacting mutant proteins of interest which retain adjuvant activity - wherein toxin-producing bacteria are treated with a mutagenizing chemical agent and toxins produced by resulting mutant strains are searched to find proteins of interest. For example, a cross-reacting protein (cross-reacting material; CRM), which showed low toxic activity and was reactive to anti-pertussis toxin antibody, was found in culture medium of a pertussis toxin-producing bacterial mutant strain. This mutant toxin is immunogenic and can be a candidate for the pertussis vaccine antigen (Y. Sato et al., Dev. Biol. Stand. 73, 93-107, 1991). In addition to this, there is a possibility that the mutant toxin might be used as the adjuvant with low toxic activity. However, the degree of toxic activity and adjuvant activity of the mutant toxin has not been reported.

Cross-reacting protein of diphtheria toxin, CRM-197 (DT-G52E) (T. Uchida et al., J. Biol. Chem. 248, 3838-3844, 1973; G. Giannini et al., Nucleic Acids Res. 12, 4063-4069, 1984), and a cross-reacting protein of pseudomonad enterotoxin A, CRM-66 (H462Y) (S.J. Cryz et al., Rev. Infec. Dis. 5 Suppl. 5, S992-S997, 1983; S. P. Kessler et al., J. Biol. Chem. 267, 19107-19111, 1992) were reported as mutant toxins which substantially lack ADP-ribosyltransferase activity. In these mutant toxins, the ADP-ribosyltransferase activity was used as the only marker for the toxic activity of these mutant toxins, and thus there is no denying of the existence of residual toxic activity generated by other mechanisms. Thus, it is possible that these mutants have other toxin activities. In addition, it was reported that when chemically linked to a polysaccharide antigen, although having adjuvant activity, CRM-197 (DT-G52E) did not stimulate sufficient antibody titers, and that consequently it required the use of additional second adjuvant (G. S. Bixler et al., Adv. Exp. Med. Biol. 303, 185-190, 1991; D. M. Granoff et al., Vaccine 11 Suppl. 1, S46-S51, 1993).

### (3) Preparation of attenuated mutant toxins by genetic engineering:

A method for constructing recombinant toxins, which still retain adjuvant activity, - wherein the toxic activity is reduced by artificially altering one or more amino acid residues of the toxin molecule utilizing gene recombination techniques. As examples, WO98/18928 discloses a subunit A of an *E*, *coli* heat labile toxin (LT-A), wherein at least amino acid Ala-72 is mutated;WO98/4237 discloses a detoxified mutant of a bacterial ADP-ribosylating toxin such as LT-K63, LT-R72, CT-S109, AND PT-K9/G129; WO95/34323 and WO95/17211 disclose genetically-detoxified pertussis toxins including those containing mutation at Glu-129"; recombinants of *E*. *coli* heat-labile toxin, wherein the arginine residue (Arg/R) at amino acid position 7 or 192 from the N-terminus was substituted with a lysine residue (Lys/K) (LT-R7K, M.T. De Magistris et al., Dev. Biol. Stand. 92, 123-126, 1998) or with glycine residue (Gly/G) (LT-R192G, B. L. Dickinson et al., Infection Immun. 63, 1617-1623, 1995), were reported to substantially lack the toxic activity but still retain the adjuvant activity.

However, according to experiments of an other research group, the toxic activity was not negligible in the above-mentioned recombinant toxins. Further, the recombinant proteins were unstable as compared with the natural one. As a result, larger amounts of the recombinant protein (relative to that of the natural one) must be used to raise antibody titer to the same level as that given by the natural one (K. Komase et al., Vaccine 16, 248-254, 1998; C. C. R. Grant et al., Infect. Immun. 62, 4270-4278, 1994).

When a toxic activity is caused by multiple activities, the reduction of any one of the activities can result in the manifestation of the other activities. The attenuation of toxin can be associated with this type of phenomenon, and therefore the marker of toxic activity should be selected carefully. As described above, attenuated adjuvants, which have been reported so far to exhibit very low toxicity, still have problems to be solved in regard to adjuvant activity, safety, stability, and productivity. Further, many screening steps must be repeated to achieve reduction of toxic activity and preservation of adjuvant activity, which are contrary subjects, by amino acid substitution. Therefore, it is a time-consuming work.

### Disclosure of the Invention

An object of the present invention is to provide an attenuated toxin, that has substantially no toxic activity but still has the excellent adjuvant activity of the toxin, as a new adjuvant.

Another object of the present invention is to provide a vaccine using one or more adjuvants of the present invention, that is safe and effective and that can be obtained by relatively simple and convenient procedures. More specifically, the object is to provide a vaccine that can be used in reduced doses, or can be used without loss of its immunogenic activity when administered by a vaccination route other than injection.

The present inventors have studied methods to reduce the toxic activity of a variety of toxins and found out that an attenuated toxin which can be prepared by a chemical treatment has substantially no toxic activity, but still has sufficient adjuvant activity of the levels to meet the criteria of practical application. The present inventors have further verified that high level of safety is expectable for this attenuated toxin and completed the present invention.

The objectives mentioned above can be attained by the present invention as characterized in the claims.

The present invention also relates to the use of an attenuated toxin as an adjuvant. The present invention further relates to the use of an attenuated toxin in the production of vaccine preparations.

The toxin composing the adjuvant of this invention is a formalin attenuated toxin that has substantially no toxic activity but still has the activity to enhance immunity. "Having substantially no toxic activity" is defined herein as having toxic activity that is less than one-two thousandth relative to that of the natural toxin. The activity of the representative bacterial toxins cholera toxin and *E*. *coli* heat-labile toxin can be compared by, for example, using the Y-1 cell morphological transformation test activity as an index. That is, for example, when the activity is reduced to one-two thousandth, then it means that the toxic activity is reduced to one-two thousandth.

Some technical papers so far reported described that ADP-ribosyltransferase was not detectable in various attenuated toxins. However, it may be improper to evaluate the toxic activity without any quantitative comparison, because the criterion is unclear in such evaluation. Further, it is problematic because it does not allow comparison of results of two papers. In the present invention, a value of one-two thousandth of the natural toxic activity was adopted as an index in order to avoid possible confusion. Even when the toxin is of bacterial origin like cholera toxin or *E. coli* heat-labile toxin, having a strong physiological activity, the high level of attenuation, wherein the toxicity is reduced to one-two thousandth that of the natural toxin, improve, to a great extent, the safety of adjuvants when injected to a human body, while retaining the adjuvant's immuno-stimulating activity.

In addition to the ADP-ribosyltransferase activity, the results obtained from bioassays utilizing physiological responses of animal cells or experimental animals may also be used as an index of toxic activity, allowing the quantitative comparison.

In any case, it is preferable to verify that the toxicity is reduced to at least one-two thousandth that of the natural toxin by comparing the relative activities using an index that sensitively reflects the activity of toxin. Further, even if a sensitive index is used, it is also desirable in terms of safety to evaluate the toxic activity by the combined use of multiple indexes, each reflecting different mechanisms, because many toxins have pleiotropic physiological activities. Particular toxins and their activities are specified later. The toxic activity of the attenuated toxin of the invention is typically reduced to at least one-two thousandth that of the natural toxin, and is reduced to at least one-ten thousandth in preferred embodiments.

Attenuation of toxins can be attained by conventional methods. For example, chemical treatments and physical treatments can be used as techniques for the attenuation of toxins. The attenuation treatments can result in various structural changes, such as irreversible changes in three-dimensional structure, dissociation of subunits, or fragmentation of peptides. However, the adjuvant activity can be maintained in the toxin by retaining the above-mentioned three types of amino acid residues (e.g., serine, glutamic acid and lysine) in it. With such known methods, the condition where the toxic activity becomes at least one-two thousandth that of the natural toxic activity, as measured by any of the above-mentioned indexes representing the toxic activity, can be determined empirically. Specific conditions of attenuation are described later in detail.

The toxins of the present invention, specific examples of which are indicated later, must retain serine residues, glutamic acid residues and lysine residues contained in the amino acid sequence of the natural toxin. A preferred toxin of the present invention is a natural toxin. A high adjuvant activity is expectable; moreover, the steps required for screening mutants may be avoided by selecting a natural toxin.

In another embodiment, the toxin of the present invention can be a mutant which has the amino acid sequence of the natural toxin, whereinone or more amino acid residues are substituted, deleted, inserted, and/or added, so long as the amino acid residues that are important for the adjuvant activity are retained. The mutant may include not only mutations in its amino acid sequence but also mutations in sugar residues, which are other constituents of the toxin.

Functional groups of amino acid residues are important for protein function. For example, serine residue and threonine residue containing an OH group, glutamic acid residue and aspartic acid residue containing a COOH group, and lysine residue and arginine residue containing an NH₂ group, often form the active center and play important roles in the expression of activity. Just as in the cases of many enzymes and functional proteins, it is believed that OH groups, COOH groups, NH₂ groups, and such in the amino acid residues forming the toxin molecule are closely associated with the expression of biological activities including, for example, enzyme activity, activity of enhancing immunity, activity of binding to a receptor, etc. Furthermore, the number of amino acids may be another index showing the importance of OH group. For example, the total contents of the three types of amino acids, namely serine, threonine, and tyrosine residue, each of which contains an OH group, in the polypeptides of cholera toxin, heat-labile toxin of human-type *E*. *coli*, and pertussis toxin are about 18%, 22% and 20%, respectively. The contents suggest the high possibility of their involvement in the active center. Accordingly, a recombinant mutant toxin that contains deletions or substitutions with other amino acids of these amino acids closely related to the active center may easily cause remarkable changes in its three-dimensional structure. Thus, it is highly possible that it loses not only toxic activity but also the activity of enhancing immunity simultaneously. As an example supporting this proposition, the present inventors have observed that in a recombinant mutant of *E*. *coli* heat-labile toxin, in which a lysine residue was substituted for the glutamic acid residue at amino acid position 112 from the N terminal end, while the toxic activity was reduced to about 1/2,860, the activity of enhancing the immunity was lost (K. Komase et al., Vaccine 16, 248-254, 1998).

Based on these findings, the present inventors selected three amino acid residues, namely serine residue, glutamic acid residue, and lysine residue, from amino acid residues having the above-mentioned three types of functional groups as being important amino acid residues for adjuvant activity. The importance of these amino acid residues for the activities of a variety of toxins has been reported previously.

Previous reports on the change of toxic activity associated with the substitutions of these amino acid residues are summarized in Table 1. Those reports described in Table 1 as having negative activity are reports in which substantially no residual toxic activity was detected in the selected condition of measurement, but in which no quantitative comparison was carried out. Adjuvant activity was detedted in *E*. *coli* heat-labile mutant toxin but the activity of enhancing immunity was not sufficiently high. Further, 10 times as much as natural cholera toxin was required for a mutant cholera toxin to give the same level of activity of enhancing immunity as natural cholera toxin. The indication "and others" means that other amino acid residues in addition to the residues indicated were also substituted. As shown, glutamic acid residue and serine residue are important residues that are often targets to be substituted with other amino acid residues. However many reports describe that such substitutions frequently result in loss or declines of the adjuvant activity.

In the present invention, amino acid residues required for the maintenance of sufficient adjuvant activity includes, in addition to these important amino acid residues that are targets for substitution, lysine residue containing an NH₂ group, which is a representative of basic amino acids. Lysine residues play an important role in the attenuation of toxin. For example, it is believed that in the formalin treatment, formalin attacks the εNH₂ group of lysine, and the formation of a Schiff base at that site results in changes of three-dimensional structure, and thus the attenuation is achieved as a consequence. Accordingly, when lysine residues are retained, it is possible to readily attain a high degree of attenuation in which the toxicity is reduced to at least one-two thousandth that of the natural toxin.

**Table 1**

| type of toxin | substitution of amino acid residues (reference) | alteration of toxic activity and adjuvant activity | | |
|---|---|---|---|---|
| *E. coli* | position 112 Glu Lys(1) | Y: | 1/2860 | |
| heat-labile toxin | position 63 Ser Lys(2) | A: | negative | |
| | | | | |
| cholera toxin | position 63 Ser Lys(2) | A: | negative | ± |
| | position 61 Ser Phe(3) | C: | 1/1000000 | ++ |
| | position 112 Glu Lys(4) | C: | 1/1000000 | ++ |
| pertussis toxin | position 129 Glu Gly and others(5) | Y: | 1/1000000 | |
| diphtheria toxin | position 162 Glu Lys and others(6) | A: | negative | |

| | | | | |
|---|---|---|---|---|
| Marker of toxic activity A: ADP-ribosyltransferase activity C: spindle-forming test with CHO cells Y: Y-1 cell morphologic transformation test Study reports (1) Komase et al., Vaccine 16, 248-254, 1998 (2) G. Douce et al., Infec. Immun. 65, 2821-2828, 1997 (3) S. Yamamoto Proc. Natl. Acad. Sci. USA, 94, 5267-5272, 1997 (4) S. Yamamoto J. Exp. Med. 185, 1203-1210, 1997 (5) M. Roberts et al., Infec. Immun. 63, 2100-2108, 1995 (6) T. Uchida et al., J. Biol. Chem. 248, 3838-3844, 1973 | | | | |

However, a highly attenuated toxin, of which toxicity is reduced to at least one-two thousandth or, more preferably, at least one-ten thousandth that of the natural toxin and having a strong activity of enhancing immunity, is not predictable from the previous reports.

For example, the cholera A subunit is thought to be responsible for the major toxic effect of cholera toxin. It has been expounded that the toxic activity is expressed when the strong ADP-ribosyltransferase activity of A subunit disturbs the balance of intracellular level of cAMP concentration in the cell permeated by the toxin. The toxic activity of *E*. *coli* heat-labile toxin is also expressed by the same mechanism. A paper was published, wherein it analyzed correlation between ADP-ribosyltransferase activity and the adjuvant activity of a recombinant of *E*. *coli* heat-labile toxin mutant. Based on the research result, it has been proposed that the two activities are closely related to each other and are not separable from each other (N. T. Lycke et al., Eur. J. Immunol. 22, 2277-2281, 1992). Other reports also note that recombinant mutant toxins with reduced enzyme activity were accompanied with decreases in adjuvant activity (for example, J. D. Clements et al., Vaccine 6, 269-277, 1988, test sample; *E*. *coli* heat-labile toxin; W. J. Black et al., Science 240 (4852), 656-659, 1988, test sample; *Bordetella pertussis* toxin).

In other words, in previous theories, it has been believed that a high level of reduction of toxin activity does not lead to development of adjuvants with high safety, because the reduction is associated with the decrease of the immuno-enhancing activity. And yet the attenuated toxin of the present invention demonstrates that the activity of enhancing immunity is substantially the same as that of the natural one, in some cases even when the toxic activity is reduced to at least one-two thousandth, more preferably at least one-ten thousandth, as compared with that of the natural one. These are novel findings first revealed based on the studies for long years by the present inventors.

It remains to be clarified why toxic activity and activity of enhancing the immunity are separable in the attenuated toxin. However, a similar phenomenon has been found for a variety of vaccines using toxoid as the antigen. These attenuated toxins have been known to retain immunogenicity. An explanation can be offered based on the mechanism. For example, the following explanation can be given for the adjuvant activity of the attenuated cholera toxin. Since the expression of toxic activity (for example, ADP-ribosyltransferase) requires a specific three-dimensional structure formed by a appropriately large-sized oligopeptide, even a minor change of three-dimensional structure results in loss of the activity. On the other hand, the activity of enhancing immunity can be expressed by the whole toxin molecule or by a smaller peptide thereof, and the activity might be maintained with minor changes in the primary structure of the toxin protein.

It has been known that cholera toxin evokes various immuno-enhancing reactions when acting as an adjuvant. For example, (1) it enhances the permeability of antigen. It binds to a receptor and activates helper T cells. It stimulates the proliferation of type-1 and type-2 helper T cells. Further, (2) it enhances the production of a variety of cytokines, for example, interferon-gamma, and interleukins 1, 4, 5, 12, etc. There is a possibility that in these immune responses, the toxin molecule effects without any structural changes thereof in the case of (1), and the toxin molecule digested to peptide fragments show the effects in the case of (2). However, details of this remain to be resolved.

An antigen protein is processed to peptide fragments in series by the action of proteosomes. The resulting peptides are presented by antigen-presenting cell. The presentation stimulates the subsequent series of immunoreactions: for example, the activation and proliferation of helper T cells, the production of a variety of cytokines, antibody production by B cells, and the like. There is a possibility that a similar mechanism to that described above works for the expression of adjuvant effects of toxin.

The above-described possibility is a hypothesis at present. While the toxic activity is mainly due to a single mechanism, the adjuvant activity is exhibited by multiple mechanisms; therefore, it can be presumed that the attenuated toxin still retains the activity of enhancing immunity even after losing the toxin activity.

### Toxin:

Attenuated toxins which constitute the adjuvant of the present invention are derived from natural toxins produced by an organism such as bacterium, fungus, plant or animal. A bacterial toxin is a particularly preferred toxin to be used as the adjuvant of the present invention. It is easy to produce bacterial toxin on a large scale and thus economically advantageous. Further, toxins derived from bacteria include, but are not limited to, toxins that have excellent adjuvant activity, e.g., cholera toxin. These include single proteins and conjugated proteins. Specifically, in the context of the present invention, the term "toxin" refers to a proteinous substance having toxic activity, which is produced by an organism in nature or a fragment or a subunit thereof. However, in the context of the present invention, the term "toxin" does not include low molecular toxins with a molecular weight less than or equal to 500 with no expected adjuvant activity. Further, toxin as defined in the present invention comprises neither toxic substances (such as poisons), which are comprised in a part of compounds synthesized by organic chemistry, nor toxic heavy metals.

The natural toxins contemplated by the present invention include the following:
1. Cholera toxin and heat-labile toxin of pathogenic *E*. *coli.*

Like pigments, antibiotics, and such, toxins are so-called "secondary metabolites". Original physiological roles of toxins in toxin-producing microorganisms, plants and animals often remain unclear. Toxin production, as a characteristic nature of secondary metabolite production, shows strain-specificity (i.e., is specific to each individual in plant and animal) and not all organisms belonging to a species are capable of producing the toxin. The frequency among strains of the same species to produce the same toxin is not constant and depends on the toxin. The same toxin can be produced by a sub-species or a related species. Multi-component toxins, with toxins having partially altered structure to a small degree, are often produced by wild-type strains, related strains and artificially mutated strains.

Thus, a mixture of multiple toxins can be used as the adjuvant of the present invention. Further, when a toxin molecule consists of multiple subunits, one or more subunits that are required for the adjuvant activity can be selected and used as the adjuvant of the invention. Toxins consisting of multiple subunits include, but are not limited to, cholera toxin, pertussis toxin, diphtheria toxin, heat-labile toxin of pathogenic *E*. *coli*, and canditoxin, etc. The subunit from a toxin, *per se,* generally exhibits adjuvant activity (JP-A Hei 2-243633). Accordingly, toxin of the invention can be such a subunit.

Further, toxins to be utilized as the adjuvant of the invention can be a mutant of the natural toxin. The mutant may have a modified structure in regard to the amino acid sequence, sugar chain and others of the natural toxin. Mutant toxins can be produced, for example, by using artificial mutant strains generated from toxin-producing wild-type or related strains by treating them with mutagenizing agents. Such a toxin is herein referred to as "artificial mutant toxin". The mutant toxin can be also produced by recombinant cells, to which mutant toxin-producing capacity has been given by utilizing gene recombination techniques. The latter is herein referred to as "recombinant mutant toxin".

Because a mutant toxin has a partially altered structure as compared with that of the natural molecule, it generally loses some of the original properties of the toxin as a result of such structural changes. However, there are cases in which the activity of enhancing immunity is retained. When the toxic activity of mutant toxin is sufficiently low, e.g., reduced by a factor of at least one-two thousandth relative to that of the natural one, it can be used as an attenuated toxin for the adjuvant of the present invention provided its activity of enhancing immunity is verified. Otherwise, the mutant toxin is further attenuated by using any of the above-mentioned chemical and physical treatments to give rise to a usable adjuvant. It is preferable that the mutant toxin is stable under the conditions where the toxin-containing vaccine is to be used. Further, mutants wherein the restoration of toxic activity is limited or negligible are preferable. "Restoration of toxin activity" refers to a phenomenon in which the toxic activity, which has been reduced greatly by a variety of treatments, is recovered as time progresses after the treatment.

There is no particular limitation on regions where the structures are to be changed in an artificial mutant toxin, so long as the specified three types of amino acid residues remain unchanged and the mutant still has the adjuvant activity. For example, such structural alteration is exemplified by one or more alterations of amino acid residue of oligopeptide moiety, sugar residue of oligosaccharide moiety, organic acid moiety, and such in the toxin molecule.

It can be difficult to artificially choose an amino acid residue or sugar residue to be mutated in the artificial mutant. Therefore, in general, it is difficult to select the type of mutation *a priori.* However, the reduction of toxic activity and the activity of enhancing immunity are easily verified during research and therefore mutants that meet the requirements of the present invention can be obtained. Further, it has the advantage that mutants with alterations of unexpected amino acid residues can be isolated.

On the other hand, it is possible to choose amino acid residues or sugar residues to be mutated and to intentionally introduce a desired mutation in regard to the recombinant mutant toxin. Specifically, such alterations include one or more alterations of amino acid residue of oligopeptide moiety, sugar residue of oligosaccharide moiety, organic acid moiety, and such in the toxin molecule. The adjuvant of the invention also includes oligopeptide fragments such as peptide fragments playing an important role in the expression of the immuno-potentiating activity of enhancing immunity.

### Production of natural toxin and mutant toxin:

Natural toxins can be obtained as the products of wild-type strains. Alternatively, the toxins can be obtained as the products of a variety of artificial mutant strains such as overproducing strains. Or the toxins can be obtained as the products of recombinant cells, in which the genes of the wild-type strain and such from the same species or a different species, have been introduced by genetic engineering. Further, the production of the toxins can be conducted by procedures of chemical synthesis.

Bacterial toxins utilized in the present invention can be extracted, separated, purified and produced, for example, using a culture of toxin-producing bacterium as a starting material and combining conventional methods. In regard to cholera toxin, an exemplary method is as follows. Toxin-producing cholera bacterium (*Vibrio chorela*) is cultured at 36°C for 18 hours. The culture liquid is used as a culture seed for large-scale cultivation on agar plates or liquid culture media. After the culture is complete, the culture supernatant (in the case of liquid culture) is concentrated by ammonium sulfate precipitation or ultrafiltration. The toxin is then purified using one or more conventional methods such as column-chromatography using Sephadex and such, ultracentrifugation, gel electrophoresis, etc. The toxic activity in a sample is assayed during cultivation or purification by the procedure described later.

In regard to pertussis toxin, it is also described an exemplary production method as follows. A pertussis bacterium (*Bordetella pertussis;* Tohama; phase I) is cultured in Bordet-Gengou medium to give a culture seed. The seed is transferred into Cohen-Wheeler medium and cultured for 48 hours and the culture supernatant is used as a starting material. The culture supernatant is loaded onto a column of hydroxyapatite. The column is washed with 0.1 M phosphate buffer (pH 7.0), and subsequent elution is performed with 0.1 M phosphate buffer (pH 7.0) containing 0.5 M NaCl. The toxin is further purified by column chromatography, using CM-Sepharose or other carriers on which adequate affinity ligands are immobilized. The toxic activity of a sample is assayed during cultivation or purification by conventional methods.

In addition, it is described toxins produced by mushrooms that can be extracted and purified from mushrooms that grow naturally or that are cultivated. A illustrative method for producing animal toxin is as follows: a toxin-producing animal, for example a snake, is bred for the toxin. The toxin is collected from the toxin-producing organ in the mouth. As the matter of course, if commercially available, the toxin may be purchased from the suppliers. Production processes for the toxin and quality thereof can be variously altered as far as the alterations meet the standard for the corresponding biological preparations and are in accordance with related laws and regulations.

An artificial mutant toxin can be produced by an artificial mutant strain derived from toxin-producing wild-type-stain. The artificial mutant strain is generated from the wild-strain by the treatment with a mutagenizing agent, for example. Known methods of mutagenesis include, but are not limited to, chemical treatment, using chemical agents such as N-methyl-N'-nitro-N-nitrosoguanidine, nitrosourea, nitrogen mastard, etc., and physicochemical treatments, such as ultraviolet light irradiation, radiation exposure with cobalt-60 or the like, heating, etc. Culture of artificial mutant strains, purification of toxins from the mutant strains, and verification of the activity thereof are carried out essentially in the same manner as for the wild-type strains.

A recombinant mutant toxin can be produced by recombinant cells. Methods for the production of such cells are conventional in the art. The following is an illustrative example of a method for producing a recombinant heat-labile toxin mutant of *E*. *coli*. A toxin-overproducing recombinant strain is prepared from a strain producing heat-labile toxin separated from patients (*Escherichia coli* 1032 strain), for example, by using the method described by Tamura et al. (S. Tamura et al., Vaccine 12, 1083-1089, 1994). As the first step, separation treatment is carried out to obtain a plasmid (65 kbp) containing both genes encoding heat-labile toxin and thermostable toxin. The plasmid is treated with restriction enzymes and such to cut out the heat-labile toxin gene (6.7 kbp). The resulting fragment is ligated into a plasmid, such as pBR322 or the like. The fragment is amplified by PCR or the like. The heat-labile toxin genes are then ligated into other expression vectors. Subsequently, site-specific mutagenesis is performed on the vector, by the method described by Ho et al. (S. N. Ho et al., Gene 77, 51-59, 1989) for example. The fragment of interest is then amplified by PCR method, using a pair of selection primers in order to give a recombinant *E. coli* strain producing recombinant mutant toxin. In the next step, the bacterial strain is cultured according to the method described by Clements et al. (J.D. Clements et al., Infect. Immun. 24, 760-769, 1979), for example. The protein of interest is purified from the culture supernatant by ultrafiltration, ammonium sulfate precipitation, agarose gel chromatography (elution with a buffer containing galactose) and others. It is thus possible to obtain recombinant mutant toxins derived from the heat-labile toxin of *E*. *coli* that is infectious to human. Alternatively, natural toxins can be obtained when the site-specific mutagenesis is not conducted in the process described above.

It is possible to replace amino acid residues with other residues at desired positions in the amino acid sequence of a toxin, for example, by using site-specific mutagenesis as described above. On the other hand, specific amino acid residues, for example a glutamic acid residue, may remain unchanged at particular positions. Sugar residues can also be treated basically in the same manner. The mutants obtained are screened to select those having adjuvant activity, which selection yields the mutant toxins of the invention. Each mutant may exhibit the toxic activity at any level. In other words, when the adjuvant activity is satisfactorily maintained but the level of toxic activity of the mutant is comparable to that of the natural one, then the mutant toxin can be used as the adjuvant after proper attenuation treatment as used for the natural one. Otherwise, if the adjuvant activity is satisfactorily maintained and the level of toxic activity of the mutant is preferably low, then the mutant toxin can be used as the adjuvant of attenuated toxin without any modification or after subjecting to some attenuation treatment. Although trial-and-error experimental approaches are required to obtain the mutants, such approaches generally have the advantage that the resulting recombinant mutant toxins hardly revert to the original toxic activity.

### Method for attenuating toxin:

Before using the adjuvants of the invention, one must first verify whether or not the toxins produced by the wild-strain, artificial mutant strain, or recombinant mutant strain have the activity of enhancing immunity after being attenuated. The toxins can be attenuated by formalin. Examples of suitable attenuation methods are described below. The term "attenuation" means herein that the toxic activity is reduced by using any means and it should not be construed as being limited to the examples. However, these methods are convenient and reliable to provide the effect of treatment.

All of these attenuation methods are publicly known methods used for converting to toxoids. In the present invention, however, it is required to attain high levels of attenuation in which toxic activity is reduced to one-two thousandth. Accordingly, more extreme conditions are required for the attenuation in the present invention, although the methods to be used here are based on the same principles as the publicly known methods. Specifically, for example when cholera toxin is treated with formalin, temperature for the treatment is 5 to 30°C (ordinary 5°C), the concentration of formalin is 0.5 to 0.8% (ordinary 0.3%), and the duration of treatment is 20 to 70 days (ordinary about 7-14 days), or the like.

### [1] Chemical method

The toxin is dissolved at an adequate concentration in a buffer. While the pH is adjusted to be within a range where the toxin is stable (typically, pH 5 to 8), an agent, for example, formalin, glutaraldehyde, phenol, iodine, acid anhydride, or a detergent such as bile acid with the adequate concentration (typically, 0.01 to 1.0%) is added stepwise. Then the solution is incubated at an adequate temperature (for example, 5°C). After incubation, the agent is removed by any suitable method such as dialysis. The residual toxic activity is then assayed. If desired, the treated toxin is incubated again at a temperature at which the attenuated toxin will be used practically, for example, at 37°C confirm that the attenuated toxin does not revert to toxicity. The attenuated toxin of interest is thus yielded. This method is based upon the known method widely used for the toxoid preparation, and is also suitable for large-scale production. However, it is preferable to use a method with which the toxic activity does not restore. There are some conventional methods used for this purpose. Exemplary methods include but are not limited to (1)the simultaneous addition of lysine in a quantity corresponding to the concentration of the agent for the attenuation treatment, for example, formalin, and (2) the performance of a reduction treatment \ after the attenuation.

### Method for verifying residual toxin activity

Residual toxic activity can be assayed by any conventional method (see, for example, "Handbook for vaccination," Ed. Research Association, National Institute of Health of Japan, Maruzen, 1994; "Protein toxin," 1st and 2nd volumes, Ed., R. Tamura et al., Kodansha Scientific, 1972, etc.). The methods can be categorized as follows: enzyme activity assays, physiological-response assays using animal cells, physiological-response assays using animals, and evaluation methods utilizing survival and death of experimental animals, etc. The specific assay method to be used depends on the type of toxin. The following examples describe representative activities as marker for selecting such a method. Specific procedures for these assays are known. The comparison of toxic activity is not limited to these indexes, but as the matter of course the comparison of toxin activities before and after the attenuation should be carried out using the same index.

### (Bacterial toxin)

Cholera toxin, heat-labile toxin of pathogenic *E*. *coli*
ADP-ribosyltransferase activity
Accumulation of cAMP
Y-1 cell morphologic transformation
Weight loss in mouse
Elongation of CHO cell

In assaying toxin activity, even when the residual toxic activity is not detected by one method, a different result can be obtained by an alternate method. For example, there was a case where the toxic activity was detected by Y-1 cell morphologic transformation test although substantially no residual activity of attenuated cholera toxin was recognized by ADP-ribosyltransferase activity (for example, K. Komase et al., Vaccine 16, 248-254, 1998). In another case, although ADP-ribosyltransferase activity was substantially not detectable in a recombinant mutant of *E*. *coli* heat-labile toxin in which the amino acid residue was replaced with lysine residue at amino acid position 7 from the N terminus, the attenuated toxin induced diarrhea. In other words, the toxic activity of *E*. *coli* heat-labile toxin can be represented by ADP-ribosyltransferase activity, but there is a second minor toxic activity that is expressed by a different mechanism. It is thought that when the first activity is markedly reduced in the recombinant mutant, then the second toxic activity increases relatively and as a consequence the second one becomes detectable. In general, assay methods with living cells or living bodies tend to show higher sensitivity in detecting toxic activity. The above-mentioned mechanism is considered to be involved in this detection.

Accordingly, in general, the comparison of toxic activity can be performed more reliably by using a sensitive method based on the response of living cells or living bodies. It is also preferable to evaluate the residual toxic activity by multiple assaying methods. In Examples described herein, the residual toxic activity of cholera toxin was evaluated by the combined use of an assay for receptor-binding activity, a Y-1 cell morphologic transformation test, a mouse foot swelling test, and a method involving the intraperitoneal administration to a mouse. Enzyme immunoassay (ELISA) using anti-toxin antibody is also a useful index representing the toxic activity. With any method, the most important thing is to reduce the toxic activity to at least one-two thousandth that of the natural toxin, in principle, based on an index that directly reflects the toxic activity.

The adjuvant of the invention comprises the above-mentioned attenuated toxin alone or in combination with a preservative, stabilizer, and/or a conventional adjuvant. In general, the safety of an adjuvant is judged by repeatedly measuring safety. In addition, it is preferable to evaluate the safety by adopting a method using a living cell or living body as one of the indexes. As a matter of course, a conventional safety test, in which acute toxicity is examined by administering the adjuvant into experimental animals, should be conducted. Further, the absence of irritability on the skin and mucous membrane is an important safety index for oral administration, percutaneous administration and intranasal administration.

### Adjuvant activity:

The present invention utilizes, as the adjuvant, an attenuated toxin in which the toxic activity is formalin-attenuated to at least one-two thousandth that of the natural toxin, as determined by a particular attenuation method, and in which the adjuvant activity is maintained. The adjuvant effect can be maintained at a high degree in the toxin by retaining the three types of amino acid residues that are specified in the summary above, namely serine, glutamic acid, and lysine. However, it is necessary to test whether or not the adjuvant activity is reduced during the process of treatment to reduce the toxic activity. The adjuvant activity can be verified by immunizing an animal with a substance to be compared for the activity, together with an immunological antigen, and then by observing the increase in titer of antibody against the immunological antigen. It is possible to compare the adjuvant activity by using a uniform condition like using animals of the same line and using identical immunological antigen.

### Usage pattern:

There is no particular limitation on the usage pattern for the attenuated toxin of the present invention as an active ingredient in a vaccine. In other words, the adjuvant can be used with various known appropriate usage patterns. For example, the adjuvant may be a physically mixed preparation or a complex chemically linked with an antigen protein. In addition, the adjuvant can be incorporated together with a vaccine in a carrier such as liposome.

The attenuated toxin of the invention can be used concurrently together with one or more conventional adjuvants. Example 5 shows the combined use of the attenuated cholera toxin adjuvant of the present invention with *E*. *coli* heat-labile toxin B subunit that is a conventionally known adjuvant. When used together with *E*. *coli* heat-labile toxin B subunit, the attenuated toxin adjuvant elevated the immunological activity in mucous membrane and further exhibited a synergistic effect. Those skilled in the art can readily find a preferable combination of adjuvants using routine experimentation, using publicly known methods. Based on the result obtained, it is possible to reduce adverse side reactions and enhance the immunoreactivity, for example, by reducing the amount of antigen or the other adjuvant.

### Vaccine:

New vaccine preparations are provided by utilizing the adjuvants of the present invention. A vaccine preparation in the context of the present invention comprises the inventive adjuvant and any immunogenic antigen, and encompasses "vaccines" in both narrow and broad senses. Specifically, the vaccines of the present invention include vaccines in the narrow sense, such as those that are effective against infection by a virus, bacterium, fungus, protozoan, or other microorganisms capable of infecting to human or other animals. Illustrative examples of such vaccines include, but are not limited to, influenza vaccine, pertussis vaccine, purified pertussis-diphtheria-tetanus toxoids combined vaccine, Japanese encephalitis vaccine, hepatitis A vaccine, hepatitis B vaccine, rotavirus vaccine, measles vaccine, rubella vaccine, mumps vaccine, measles-rubella-mumps trivalent vaccine, measles-rubella divalent vaccine, vaccine against haemophilus influenzae, etc. The vaccines also include tuberculosis vaccine, vaccine against multi-drug resistant Staphylococcus aureus (MRSA), *Helicobacter pylori* vaccine, enterohaemorrhagic *Escherichia coli* (EHEC) vaccine, salmonella vaccine, *Chlamydia* vaccine, *Mycoplasma* vaccine, AIDS vaccine, malaria vaccine, coccidium vaccine, and schistosome vaccine. Vaccines in the broad sense include those vaccines that are effective in the prevention and treatment of non-infectious diseases such as anti-cancer vaccine, vaccine of contraception, anti-gastric ulcer vaccine, anti-diabetic vaccine and anti-arteriosclerotic vaccine. Vaccines can be administered by injection, orally, percutaneously, intranasally or by other methods.

The vaccines of the present invention include various vaccines that can be categorized based on the types of methods to produce them. Specifically, the vaccines of the present invention include attenuated live vaccines, inactivated vaccines, component vaccines, vaccines using DNA, etc. The "vaccines using DNA" include both vaccines containing a DNA fragment integrated in a carrier, such as plasmid, and vaccines used in combination with ribozymes or antisense oligonucleotides, and the like. These vaccines can be used for prevention or treatment. The vaccines also include recombinant vaccines containing an antigen, which is the active ingredient of vaccine, produced in living cells engineered by gene recombination techniques. These vaccines may be monovalent vaccines or combined vaccines. Illustrative production methods and usage patterns of the vaccines are as follows.
- Influenza vaccine: a split vaccine which contains hemagglutinin (HA), neuraminidase (NA), nuclear protein (NP), matrix protein (M), or a part thereof, obtained by (a) growing viruses in embryonated eggs or in Vero cells or by other animal cell culture techniques and (b) decomposing the virus particles into respective components thereof with ether, detergent or the like and purifying the virus components, or by genetic engineering or chemical synthesis and which is administered by injection, orally, percutaneously or intranasally; alternatively, a DNA vaccine containing DNA fragments of genes encoding these proteins to be given by intranasal route.
- Pertussis vaccine: an inactivated vaccine that is obtained by culturing *Bordetella pertussis,* treating the culture supernatant or bacteria by salting-out, ultracentrifugation, and the like to extract constituents of interest, and detoxicating with formalin; alternatively, a recombinant vaccine containing *Bordetella pertussis* toxin (PT), hemagglutinin (FHA), 69K membrane protein, or a partial peptide thereof, each of which is prepared by genetic engineering or chemical synthesis.
- Pertussis-diphtheria-tetanus combined vaccine: a trivalent vaccine prepared by mixing pertussis vaccine with diphtheria toxoid and tetanus toxoid, which is administered by injection, orally, percutaneously or intranasally.
- Japanese encephalitis vaccine: a vaccine containing antigen proteins that are obtained by (a) growing viruses in the mouse brain or in Vero cells using animal cell culture techniques, (b) purifying the virus particles by ultracentrifugation or with ethyl alcohol, and (c) inactivating with formalin; alternatively, antigen proteins can be obtained by genetic engineering or chemical synthesis.
- Hepatitis B vaccine: a plasma vaccine that is obtained using blood collected from hepatitis B carriers as raw material, and separating and purifying HBs antigen by salting-out, ultracentrifugation, and others; alternatively, a recombinant vaccine containing antigen portions obtained by gene engineering or chemical synthesis.
- Measles vaccine: a live vaccine of attenuated virus that is prepared by growing the viruses in culture cells such as chicken embryonic cells or in embryonated eggs; alternatively, a vaccine containing a part of the virus or a recombinant vaccine containing the protective antigen prepared by gene engineering or chemical synthesis.
- Rubella vaccine: a vaccine containing viruses grown in culture cells, such as chicken embryonic cells or in embryonated eggs, part of the virus or the protective antigen prepared by genetic engineering or chemical synthesis.
- Mumps vaccine: an attenuated live vaccine containing viruses grown in culture cells, such as rabbit cells or in embryonated eggs, part of the virus, or the protective antigen prepared by gene engineering or chemical synthesis.
- Measles-rubella-mumps vaccine; a trivalent vaccine that is obtained by combining measles vaccine, rubella vaccine, and mumps vaccine.
- Measles-rubella vaccine; a divalent vaccine that is obtained by combining measles vaccine and rubella vaccine.
- Rotavirus vaccine: a vaccine containing viruses grown in culture cells such as MA104 cell, viruses collected from patient's feces, part of the virus, or protective antigen prepared by genetic engineering or chemical synthesis.
- *Mycoplasma* vaccine: a vaccine containing *Mycoplasma* grown in medium for *Mycoplasma*, a part thereof, or protective antigen prepared by genetic engineering or chemical synthesis.
- AIDS vaccine: a vaccine containing viruses grown in culture cells or viruses obtained from patients, part thereof, or protective antigen prepared by genetic engineering or chemical synthesis; alternatively, a DNA vaccine containing effective DNA fragments.
- *H. pylori* vaccine: a vaccine containing, as antigens, lysate of cultured *H. pylori,* or urease, heat shock protein, toxin and others separated from *H. pylori* culture; alternatively, a vaccine comprising these antigen proteins produced by genetic engineering, and administered by injection, oral inoculation, percutaneous inoculation or intranasal inoculation.

### Properties of vaccine:

The vaccines above may be provided as liquid forms or powdered forms.

When each of these vaccines is administered together with an attenuated toxin adjuvant of the present invention, the liquid form of the vaccine is often more suitable for intranasal inoculation (intranasal spray, intranasal instillation, spread, etc.) and injection. Further, the intranasal inoculation can be performed with powder spray. The vaccine preparations of the invention can also be formulated with a conventional stabilizer or preservative. An illustrative stabilizer includes, but is not limited to, gelatin and dextran of about 0.2%, sodium glutamate of 0.1 to 1.0%, lactose of about 5%, and sorbitol about of 2%. Preservatives include about 0.01% thimerosal, phenoxyethanol of about 0.5%, and about 0.1% β-propiolactone.

### Mixing ratio of attenuated toxin:

The mixing ratio between a vaccine antigen and an attenuated toxin adjuvant of the present invention can be, for example, 1:0.0001 to 1:10000 (by weight ratio) in vaccine preparations in accordance with the present invention. However, this range is merely illustrative and is not limiting on the vaccines of the present invention. A suitable ratio can be routinely selected depending on the type of vaccine. Methods required for selection are known to those skilled in the art. As seen in the Examples below, an inventive adjuvant comprising an attenuated toxin derived from a natural one in accordance with the present invention exhibits activity of enhancing immunity, the level of which is the same as that of the natural one when used at the same quantity. On the other hand, with a conventional recombinant mutant, it is often necessary to inoculate with a larger quantity to attain the same level of immuno-enhancing activity as that achieved with the natural toxin.

### Mixing procedure of attenuated toxin:

The vaccine preparation of the present invention can be prepared by combining the above-mentioned vaccine with the attenuated toxin adjuvant of the invention at an adequate mixing ratio. The preparation must be done in a strictly sterile condition. Each of the raw materials must also be prepared under a completely sterile condition. It is desirable to remove contaminated proteins, i.e., those that have no vaccine activity and that act as pyrogens or allergens, as much as possible. Methods to achieve the treatment are conventional and known to those skilled in the art. The vaccine preparation of the invention can be effective even when the vaccine antigen and the attenuated toxin of the invention are separately prepared as pharmaceutical preparations and then the two are combined with each other at the time of inoculation or the two are inoculated at about the same time.

### Vaccination method:

Any conventional method can be utilized for dosage pattern of the vaccine preparation in accordance with the present invention.

The dose is preferably about 5 to about 50 µl for intranasal inoculation to mouse; preferably about 0.1 to about 1.0 ml for the inoculation to human by intranasal administration or injection. Percutaneous inoculation can be performed by fixing the vaccine composition to a carrier and then contacting with the skin. The dose of antigen protein in a vaccine composition generally ranges from about 0.1 ng to about 100 mg per administration, preferably from about 1 µg to about 1 mg per administration. On the other hand, the amount of adjuvant to be formulated is determined depending on the type and dose of immunological antigen to be used. The carrier for the vaccine composition can be an absorptive carrier, such as lint, and a conventional carrier for applying the dose as a paste or spray.

Oral, percutaneous or intranasal inoculation is highly preferred for the vaccines exemplified below, both in terms of effectiveness of vaccine and convenience of inoculation procedure. Examples of such vaccines include, but are not limited to, influenza vaccine, pertussis vaccine, diphtheria vaccine, rotavirus vaccine, measles vaccine, rubella vaccine, mumps vaccine, *Helicobacter pylori* vaccine, enterohaemorrhagic *Escherichia coli* (EHEC) vaccine, *Chlamydia* vaccine, *mycoplasma* vaccine, coccidium vaccine, AIDS vaccine, malaria vaccine, and schistosome vaccine, etc.

These vaccines can be administered alone or in combination with other agents, by mixing more than two of them and inoculating them simultaneously, for example, like pertussis-diphtheria-tetanus trivalent vaccine or measles-rubella divalent vaccine. One of the reasons why oral and intranasal inoculations are preferred is that mucous membranes of the respiratory tract and digestive tract are important sites of early stage infection. Such inoculations can prevent infection at a stage as early as possible by activating the immunogenic system in the local mucous membrane with the inoculation of a vaccine comprising an appropriate adjuvant possessing potent immuno-stimulating activity. Further, for some vaccinations, such as vaccinations against Malaria *Plasmodium,* which are typically performed in regions without sufficient medical facilities, it is advantageous to select vaccination routes such as oral, percutaneous and intranasal inoculation routes because such vaccines can be administered without the help of medical staff such as physician or nurse. In addition, percutaneous inoculation is desirable, because the vaccination can be halted as may be demanded when adverse side effect or the like encounters.

### Brief Description of the Drawings

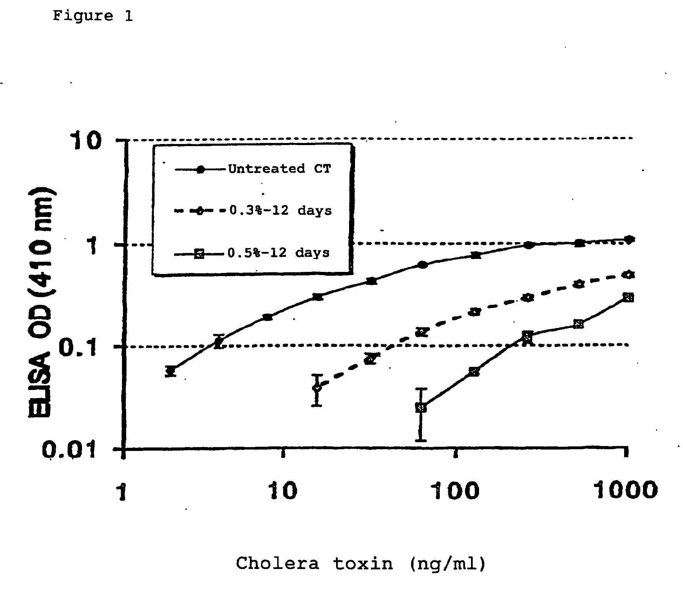

Figure 1 is a graph showing the binding capacity of attenuated cholera toxin to ganglioside GM1.
   This figure shows an example of a confirmation test for attenuation, which was conducted during the process of producing an adjuvant of the invention by attenuating cholera toxin with formalin treatment. In this figure, the ordinate axis indicates the binding capacity to ganglioside GM1, which is the receptor (O.D. at 410 nm in ELISA), and the abscissa axis indicates toxin concentration (ng/ml).

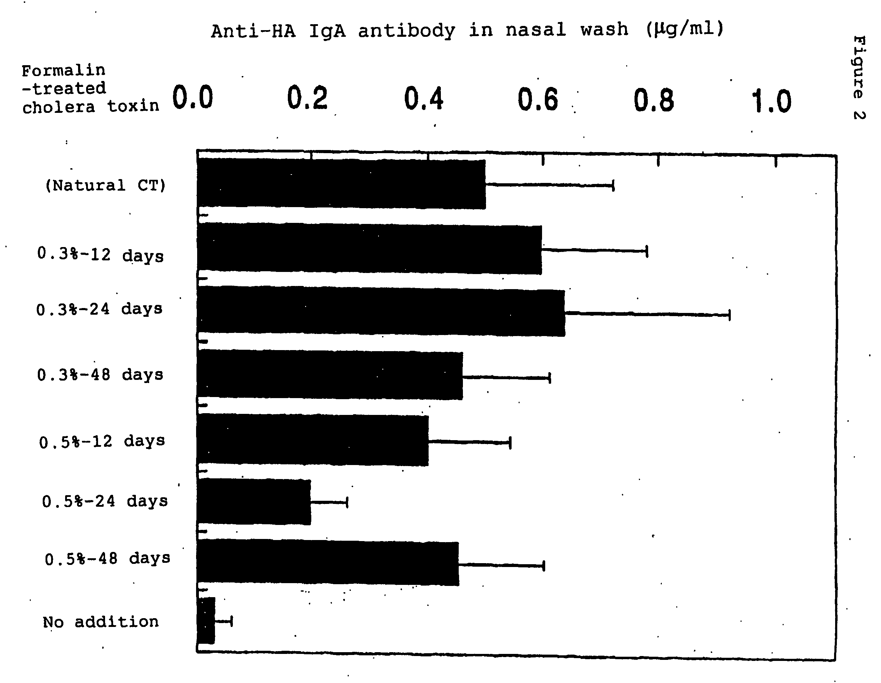

Figure 2 is a graph showing the effect of attenuated cholera toxin to enhance production of anti-influenza virus antibody in the nasal mucous membrane by the secondary response.
   This figure shows the antibody titer in nasal wash, resulting from the intranasal administration of a vaccine preparations of the present invention comprising an influenza vaccine antigen. The ordinate axis indicates attenuated cholera toxins treated under a variety of conditions and the abscissa axis indicates the concentration (µg/ml) of anti-HA IgA antibody in the nasal washes.

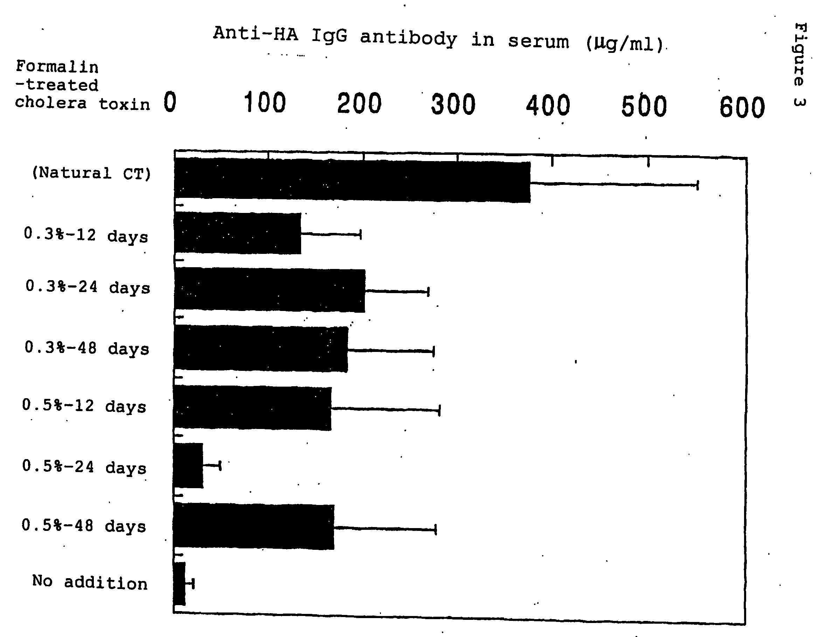

Figure 3 is a graph showing the effect of attenuated cholera toxin to enhance the production of anti-influenza virus antibody in the serum by the secondary response.
This figure shows the antibody titer in the serum, resulting from the intranasal administration of a vaccine preparation of the present invention comprising an influenza vaccine antigen. The ordinate axis indicates attenuated cholera toxins treated under a variety of conditions and the abscissa axis indicates the concentration (µg/ml) of anti-HA IgA antibody in the serum.

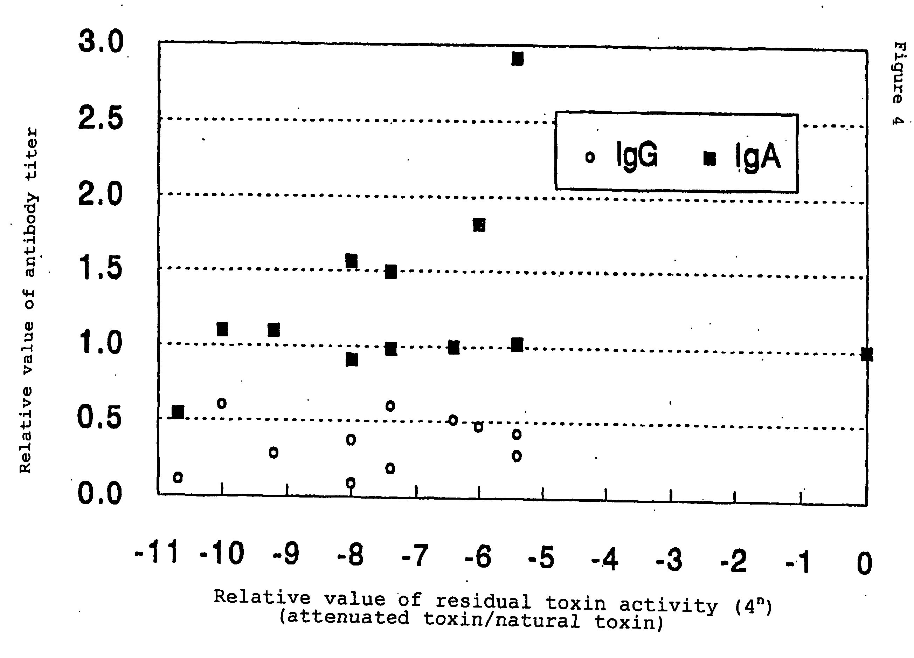

Figure 4 is a graph showing the immuno-stimulating activity of attenuated toxin.
   This figure shows the antibody titer in the nasal wash or in the serum in the secondary response, resulting from the intranasal administration of an influenza vaccine of the present invention, containing cholera toxins of which residual toxin activities are different to each other as the adjuvants. The ordinate axis indicates a relative value (□) of anti-HA IgA antibody titer in the nasal wash of the experiment group, where attenuated cholera toxin was used, to anti-HA IgA antibody titer in control group, where natural cholera toxin was used, or a relative value (□) of anti-HA IgG antibody titer in the serum of the experiment group, where attenuated cholera toxin was used, to anti-HA IgG antibody titer in control group, where natural cholera toxin was used. The abscissa axis indicates a relative value of residual toxic activity of attenuated cholera toxin used in the test to the activity of natural toxin (measured by Y-1 cell morphologic transformation test).

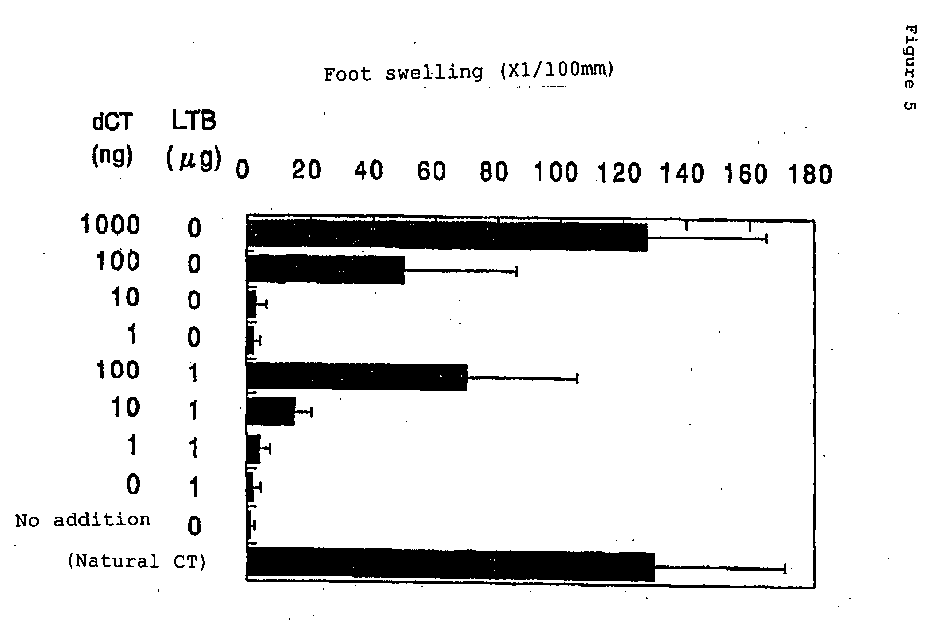

Figure 5 is a graph showing the synergistic effect of dual adjuvants.
   This figure shows immunological responses resulting from the intranasal administration of an influenza vaccine that contains the attenuated cholera toxin of the invention and a conventional *E. coli* heat-labile toxin B subunit as the adjuvants. The ordinate axis indicates the type and amount of adjuvant used in the test and the abscissa axis indicates the degree of immunological response measured by mouse foot swelling test.

### Best Mode for Carrying out the Invention

Examples of the present invention are illustrated below, but the present invention is not to be construed as being limited thereto.

### Example 1 - Preparation of cholera toxin:

The cholera toxin was produced according to the method described by Finkelstein et al. (R. A. Finkelstein et al. J. Infect. Dis., 121, Suppl., S63, 1970).

A cholera toxin-producing bacterium (*Vibrio chorela;* Inaba type 569B strain) was cultured in a semi-synthetic casamino acid medium (liquid medium containing glucose) designed by Finkelstein et al., at 30°C for 20 hours. After the culture was completed, the culture supernatant was subjected to ultrafiltration, using a filter having pores that the cholera toxin molecule (molecular weight; 86,000 Da) could freely go through. A small amount of aluminum hydroxide gel was added to the filtrate. The gel was allowed to adsorb the toxin and then was collected by centrifugation. The toxin was eluted from the gel with 10% phosphate-10% citrate (pH 7.6), while incubating at 30 to 35°C. The eluted liquid was dialyzed against an aqueous solution containing 0.1 M citrate. Then, the solution was loaded onto a column of DEAE-Sephadex and the toxin was eluted with phosphate buffer containing 0.1 to 0.2 M saline (hereafter abbreviated as PBS). Subsequently, the sample was loaded onto a column of Sephadex G-75 and the toxin was eluted with PBS. The resulting sample was subjected to gel electrophoresis. The presence of the cholera toxin only was detected as a single band. The purity was about 95%. The yield was 250 mg/100L culture broth.

### Example 2 - Attenuation of cholera toxin:

The purified cholera toxin obtained in Example 1 was dissolved in 0.01 M PBS (pH 7-8). 0.05 M lysine was added to the solution. Then to 6 aliquot of the solution formalin was added dropwise at final concentrations of 0.1, 0.3, 0.5, 0.6, 0.8, or 1.0%. The resulting solutions were incubated at 30 to 40°C for 7 to 96 days. Samples were withdrawn during the incubation. Immediately afterwards, the collected samples were dialyzed against 20 times as much volume of PBS to remove formalin. The dialyzed solution was then sterilized by filtration to give attenuated cholera toxin preparations. The time course of the activity change was investigated by using part of the samples collected during the treatment. Figure 1 shows a typical time course where the sample collected on the twelfth day of the formalin treatment was assayed by the method for measuring the residual toxic activity , utilizing a marker the ability to bind cholera toxin B subunit (a subunit having the binding activity to target cells of toxin; A subunit is responsible for the toxic activity) to ganglioside GM1, which is its receptor. The result shows that the binding ability of the attenuated toxin to ganglioside GM1 is reduced to about 1/15 and 1/100 of the natural one, when the toxin was treated with 0.3% and 0.5% formalin for 12 days, respectively.

The Y-1 cell morphological transformation test was utilized to assay the residual toxic activity of a variety of samples that were subjected to attenuation treatment under various conditions. Part of the result is indicated in Table 2. The result shows that the residual activity is reduced by a factor of about 1/1780 to about 1/114000 when the toxin was treated with 0.3% and 0.5% formalin for 12 to 96 days. Acute toxicity test was conducted in mice by using the attenuated cholera toxin prepared above. The attenuated cholera toxin, indicated in Table 2, in which the residual toxic activity was 1/7000 of the original activity or lower, did not show toxicity when administered intraperitoneally at a dose of 30 mg/kg. Accordingly, the toxic activity can be reduced to one-two thousandth or lower (using acute toxicity in mouse as marker) by treating the toxin with 0.3% formalin at 35°C for 12 days or more.

**Table 2**

| Residual toxic activity of formalin-treated cholera toxin (according to the result obtained by Y-1 cell morphological transformation test□ | | | | |
|---|---|---|---|---|
| Formalin Treatment | | Residual Toxicity (ED50) | Attenuation Rate | |
| Concentration (%) | Days | Dilution (4ⁿ) | (4ⁿ) | Approximate Value of Attenuation Magnification |
| 0.3 | 12 | 2.0±0.6 | 6.7 | 10,800 |
| | 24 | 2.3±0.6 | 6.4 | 7,130 |
| | 48 | 2.7±0.6 | 6.0 | 4,100 |
| | 72 | 3.3±0.6 | 5.4 | 1,780 |
| | 96 | 3.3±0.6 | 5.4 | 1,780 |
| 0.5 | 12 | 0.3±0.6 | 8.4 | 114,000 |
| | 24 | 0.7±0.6 | 8.4 | 65,500 |
| | 48 | 1.3±0.6 | 7.4 | 28,500 |
| | 72 | 1.3±0.6 | 7.4 | 28,500 |
| | 96 | 0.7±0.6 | 8.0 | 65,500 |
| No treatment control | | 8.7±0.6 | | 1 |

### Example 3 - Enhancing effect on the secondary production of antibody against influenza HA vaccine:

HA antigen prepared from influenza virus PR8 strain (A/Puerto Rico/8/34, H1N1 type) that had been adapted to mouse was used as an antigen for the vaccine. Attenuated cholera toxin was used as the adjuvant. The residual toxic activity of attenuated cholera toxin used was 1/1780 to 1/114000 that of the original activity (according to Y-1 cell morphological transformation test). Five Balb/c mice (6-weeks-old, female) were used for each group in the test. The mice were anesthetized with sodium amobarbital, which was administered into the peritoneal cavity. 10 µl of PBS containing 1 µg vaccine antigen and 1 µg adjuvant was given dropwise to either nasal cavity of the mouse for intranasal immunization. Four weeks later, the secondary immunization was carried out in the same manner. Two weeks after the secondary immunization, sera and nasal washes were collected from the mice.

The titer of anti-influenza virus antibody in the serum was determined based on the HI antibody titer. After bloodletting, the nasal washes were collected from the mice by perfusing the right and left nasal cavities with 1 ml of PBS containing 0.1% bovine serum albumin (BSA). The quantities of anti-HA-IgA antibody in the nasal washes and anti-HA-IgG antibody in the sera were determined by enzyme immunoassay (ELISA). Prior to the assay for anti-HA-IgA, each well of EIA plate was treated with 50 µl of HA vaccine (5 µg/ml) suspended in a coating buffer. The plate was allowed to stand for the coating at room temperature for 2 hours. The plate was then washed with PBS containing Tween-20 (hereinafter abbreviated as PBS-Tween). Subsequently, each well was coated with 100 µl of PBS containing 1% BSA and 0.1% NaN₃ to prevent nonspecific reactions. The plate was allowed to stand at 4°C overnight, and then washed with PBS-Tween. A 100-µl aliquot of adequately diluted nasal washes sample was added to each well. After several hours, the reaction solution was discarded and the well was washed with PBS-Tween Subsequently, 100 µl of alkaline phosphatase-labeled goat anti-mouse IgA α chain-specific antibody (or alkaline phosphatase-labeled goat anti-mouse IgG antibody) diluted with PBS containing 1% BSA and 0.1% NaN₃ was dispensed into the respective wells. The plate was allowed to stand at room temperature for an hour and then washed. Finally, p-nitrophenyl phosphate (1 mg/ml; Sigma Co.) dissolved in 10% diethanol amine buffer (pH 9.8) was added to each well for color development. The plate was allowed to stand at room temperature for 20 to 30 minutes, and then the coloring reaction was monitored at O.D. (405 nm) in a plate reader.

Figure 2 shows the influence of an influenza vaccine, comprising attenuated cholera toxin prepared in Example 2 as the adjuvant, on the production of anti-influenza HA IgA antibody by the secondary response in the nasal washes. When the adjuvant-free vaccine was administered intranasally, the titer of anti-HA-IgA antibody was low. On the other hand, it was observed that the titer of anti-HA-IgA antibody in the nasal washes was markedly elevated in the group given primary and secondary vaccine containing attenuated cholera toxin. The titer of antibody is comparable to that of the control in which the same dose of natural cholera toxin was used.

Figure 3 shows the influence on the production of anti-HA IgG antibody in the serum in the above-described test. The attenuated toxin elevated approximately 15 times the titer of HA antibody in the serum as compared with that with the adjuvant-free vaccine. However, the antibody titer was about half of that with natural cholera toxin used as a control. These results show that the attenuated cholera toxin is useful as the adjuvant for vaccine, for example, when the local immunity is required to be enhanced.

### Example 4 - Adjuvant activity of highly attenuated toxin:

A variety of attenuated cholera toxins, of which residual toxic activities were all less than one-two thousandth of that of the natural one, were used as the adjuvants. The same experiment as in Example 2 was repeated several times to assay the titer of anti-HA-IgA in the nasal wash and titer of IgG antibody in the blood of mice. The antibody titers determined in each assay were converted to relative values to those observed when the same amounts of natural cholera toxin were use as positive controls. The relative value is indicated as the ordinate axis and the attenuation rate of the toxin (relative residual activity to the natural one) was indicated as the abscissa axis (Figure 4). It is obvious that the majority of attenuated toxins, of which residual toxic activities are less than one-two thousandth that of the natural one (one-two thousandth □4^{-5.46}), exhibit high levels of antibody production-enhancing activity comparable to that of the same amount of natural cholera toxin. In particular, it was verified that the attenuated toxins, of which residual toxic activities are reduced to at least one-two thousandth that of the natural one, show comparable or higher efficacy as compared to that of original toxin on intranasal administration in regard to the enhancement of antigen-specific mucosal IgA formation.

### Example 5 - Use of dual adjuvant:

A cholera toxin attenuated by the formalin treatment (having a residual toxic activity of about 1/1048000 that of the natural one in Y-1 cell morphological transformation test) and *E. coli* heat-labile toxin B subunit (untreated), which toxic activity had previously been confirmed to be weak, were used as the adjuvant together. This experiment was carried out in the same manner as in Example 3. The cellular immune response to influenza virus was evaluated by the mouse foot pad test. The result is shown in Figure 5. When 1 µg of *E*. *coli* heat-labile toxin B subunit and 0.1 µg or 0.01 µg of attenuated cholera toxin were used together, the adjuvant enhanced immune response evidently much higher than that exhibited with the same amount of attenuated cholera toxin alone. The result shows that the dose of attenuated toxin can be further reduced by the method in which the attenuated toxin is used together with another adjuvant.

### Example 6 - Preparation of various attenuated toxins and enhancing effect thereof on the antibody production:

Diphtheria toxin and pertussis toxin was prepared according to a method described by the Kitasato Institute for producing diphtheria vaccine and pertussis vaccine ("Textbook of techniques for vaccine production," Kitasato Institute, 1986). Each toxin was attenuated in the same manner as shown in Example 2 and was adsorbed on aluminum gel and used in the subsequent experiments. In addition, commercial Staphylococcal a toxin (Sigma Co.) and enteritis vibrio thermostable toxin (Sigma Co.) were purchased and attenuated in the same manner as described in Example 2. The toxins were used without further modification. A recombinant mutant LTR(7)K (lysine residue is substituted for arginine residue at amino acid position 7 from the N terminus) of *E*. *coli* heat-labile toxin was prepared by a method described by Komase et al. (K. Komase et al., Vaccine 16, 248-254, 1998). Thus five types of attenuated toxins were obtained. The effect of enhancing immunity of influenza vaccine was tested according to the same method as described in Example 3, except that the attenuated toxins prepared above were used at appropriate concentrations instead of the attenuated cholera toxin in Example 3. The result is shown in Table 3, where relative value of residual toxic activity of attenuated toxin (ratio of toxin attenuated versus the natural one) is also indicated.

The result shows that both adjuvants of the invention, whether the natural toxin or the recombinant mutant toxin, exhibit activity of enhancing immunity of influenza vaccine administered by the intranasal route.

**Table 3**

| Enhancing effect of various attenuated toxins on the antibody production | | | | |
|---|---|---|---|---|
| Attenuated Toxin | Ratio of attenuation (1/4ⁿ) | Inoculation Volume (µg/mouse) | | Antibody Production HI (2ⁿ) |
| | | Toxin | Influenza HA | |
| Staphylococcal α Toxin | 6.7 | 0.5 | 2.0 | 9.0 |
| | 9.1 | 0.5 | 2.0 | 8.6 |
| Pertussis toxin | 7.9 | 1.0 | 2.0 | 11.0 |
| | 9.3 | 1.0 | 2.0 | 10.8 |
| Diphtheria toxin | 7.2 | 1.0 | 2.0 | 10.5 |
| Recombinant | 5.8 | 5.0 | 2.0 | 10.9 |
| of *E.coli* thermolabile toxin (LT-R7K) | 9.3 | 5.0 | 2.0 | 11.8 |
| Enteritis vibrio thermostable toxin | 8.4 | 0.5 | 2.0 | 9.0 |
| Chorea toxin (control) | 0.0 | 1.0 | 2.0 | 11.2 |
| No addition (control) | | 0.0 | 2.0 | < 4 |

### Example 7 - Pharmaceutical preparation containing pertussis-diphtheria-tetanus combined vaccine and attenuated cholera toxin (nasal drop):

Pertussis-diphtheria-tetanus combined vaccine was mixed with an attenuated cholera toxin (having a toxic activity of about 1/100000 as compared with that of the natural one) dissolved in PBS and sterilized by filtration. 20 µl of the solution contained the combined vaccine, of which amount corresponded to 50 µg of protein nitrogen, and 5µg of attenuated cholera toxin. A preservative (0.005% thimerosal) was added to the solution. The resulting mixture was dispensed into appropriate containers, which was used as intranasal inoculum comprising pertussis-diphtheria-tetanus combined vaccine and attenuated cholera toxin. The preparation was stored at a temperature of less than 10°C in a cool and dark place.

Vaccine prepared as above containing pertussis-diphtheria-tetanus combined vaccine and attenuated toxin was inoculated to mice of each group. The same amount of the vaccine was further given to each mouse for supplementary inoculation after 4 weeks. After 2 weeks following the last immunization antibody production was evaluated. When vaccine alone was inoculated into mice, the titers (international unit) of anti-pertussis toxin antibody, anti-diphtheria toxin antibody, and anti-tetanus toxin antibody were 2.0 units or lower, 2.0 units or lower, and 1.5 units or lower, respectively. On the other hand, the other group subjected to vaccination with attenuated toxin showed respective titers of 88.6, 61.8, and 75.5 units. The test result indicates that, in the group subjected to vaccination with attenuated toxin, the amount of produced antibodies against the respective antigens are larger than those in mice inoculated with the adjuvant-free vaccines.

### Example 8 - Pharmaceutical preparation containing tetanus vaccine and attenuated cholera toxin (percutaneous inoculum):

Tetanus toxoid was prepared as an antigen according to a method described by Kitasato Institute for producing tetanus toxoid ("Textbook of techniques for vaccine production," Kitasato Institute, 1986). Cholera toxin and attenuated cholera toxin (having a toxic activity of about 1/2500 of the natural one) were prepared by the method described in Examples 1 and 2. The two were used as the adjuvants in the test. A tetanus toxoid solution (400 Lf/ml) was mixed with a solution of either cholera toxin (4 mg/ml) or attenuated cholera toxin (4 mg/ml) at various volume ratios to give vaccine preparations (1 ml). After preparation, vaccine was immediately soaked into the cloth base (lint, about 2 cm x 2 cm) and then used for immunization. Guinea pigs (5 in each group) were immunized by the following manner. The back of each guinea pig was shaved with a hair clipper, and then some epilation cream was spread on the back. After 30 minutes, the back of the guinea pig was washed. On the following day, cloth base on which the sample had been spread was patched on the back. The cloth was fixed with an adhesive tape. The mice were bred in the usual manner. The cloth was changed with a fresh one once a week, four times in total. The first blood collection was carried out on the fourth week after the first patch was given. Six weeks after the primary immunization, the fifth was patched (booster), and then one week after that (seven weeks after the primary immunization), the second blood collection was performed.

A tetanus antibody assay kit "KAKETSUKEN" was used for assaying antibody titer in the blood. The assay result for the titer of antibody against tetanus toxin is shown in Table 4. After seven weeks, the antibody titer was found to be higher in the group given vaccine with attenuated cholera toxin than that observed in the group given vaccine without attenuated cholera toxin (control).

**Table 4**

| Test group No. | Tetanus Toxoid (Lf/ cloth base) | Toxin (mg/cloth base) | Antibody titer in blood (international unit/mL) | |
|---|---|---|---|---|
| | | | After 4 weeks | After 7 weeks |
| 1 | 100 | Attenuated cholera toxin 1.0 | 3.6 | 12.3 |
| 2 | 100 | Attenuated cholera toxin 0.1 | 2.6 | 9.2 |
| 3 | 100 | Natural cholera toxin 1.0 | 4.1 | 25.6 |
| 4 | 100 | Natural cholera toxin 0.1 | 2.6 | 14.3 |
| 5 | 100 | No addition | 2.0 or less | 3.1 |

### Example 9 - Hepatitis B vaccine-attenuated cholera toxin (injection):

Hepatitis B vaccine was combined with a fraction containing attenuated cholera toxin (having a \ toxic activity reduced to at least 1/10⁶ as compared with that of the natural one) that had been dissolved in PBS and sterilized by filtration. 1 ml of the solution contained HBs antigen, of which amount corresponded to 40 µg of protein, and 10 µg of the attenuated cholera toxin. A preservative (0.01% thimerosal) as well as a stabilizer (0.2% porcine gelatin) was added to the solution. The resulting mixture was dispensed into appropriate containers, and was used as the hepatitis B vaccine-attenuated cholera toxin injection. The preparation was stored at a temperature of less than 10°C in a cool and dark place.

The hepatitis B vaccine prepared above was inoculated into mice. The antibody production in the blood was evaluated after 3 weeks. The result of passive hemagglutination test showed that the antibody titer was 2^{3.6} units in mice subjected to the inoculation of adjuvant-free vaccine and the titer was 2^{5.8} units in mice with attenuated cholera toxin.

### Example 10 - Pharmaceutical preparation containing Japanese encephalitis vaccine and attenuated cholera toxin (injection):

Japanese encephalitis vaccine was mixed with attenuated cholera toxin (having a toxic activity of at least 1/100000 that of the natural one) that had been dissolved in PBS and sterilized by filtration. 1 ml of the solution contained inactivated Japanese encephalitis virus particles corresponding to 10^{7.0} PFU and 2 µg of attenuated cholera toxin. A stabilizer (0.2% porcine gelatin) was added to the solution. The resulting mixture was dispensed into appropriate containers, and was used as the Japanese encephalitis vaccine-attenuated cholera toxin injection. The preparation was stored at a temperature of less than 10°C in a cool and dark place.

The Japanese encephalitis vaccine prepared above was inoculated twice at a 1-week interval into mice. The antibody titer in the blood was evaluated. The titer of neutralizing antibody was 10^{1.8} units in mice subjected to the inoculation of the adjuvant-free vaccine, and the titer was 10^{2.9} units with attenuated cholera toxin.

### Example 11 - Pharmaceutical preparation containing measles-rubella vaccine and attenuated cholera toxin (nasal drop):

Measles-rubella vaccine was mixed with attenuated cholera toxin (having a residual toxic activity of about 1/28500 that of the natural one) dissolved in PBS and sterilized by filtration. 20 µl of the solution contained the respective vaccines of which amounts corresponded to 7 µg virus particles, and 2.5 µg attenuated cholera toxin. A stabilizer (0.2% porcine gelatin, 0.1% sodium glutamate, 5% lactose) was added to the solution. The resulting mixture was dispensed into appropriate containers, and was used as the nasal drop of measles-rubella vaccine-attenuated cholera toxin. The preparation was stored at a temperature of less than 10°C in a cool and dark place.

The measles-rubella vaccine prepared above was inoculated twice at a 3-week interval into mice. Then the antibody production in the blood was evaluated. The test result showed that the ELISA antibody titer was 0.18 for measles or 0.08 for rubella in mice subjected to the inoculation of the vaccine alone, and the titer was 0.34 for measles or 0.42 for rubella with attenuated cholera toxin-containing vaccine.

### Example 12 - Pharmaceutical preparation containing measles-rubella vaccine and attenuated pertussis toxin (nasal drop) :

The antibody titer in mouse blood was assayed according to the same method as described in Example 11 except that the attenuated pertussis toxin (the same as prepared in Example 6; having a toxic activity of about 1/100000 that of the natural one when observed by CHO cell morphological transformation test) was used instead of the attenuated cholera toxin used in Example 11. The ELISA antibody titer was 0.19 for measles or 0.070 for rubella in mice subjected to the inoculation of the adjuvant-free vaccine, and the titer was 0.32 for measles or 0.16 for rubella with attenuated pertussis toxin-containing vaccine.

### Example 13 - Pharmaceutical preparation containing rotavirus vaccine and attenuated recombinant E. coli heat-labile toxin (oral vaccine and nasal drop):

Rotavirus vaccine was mixed with a fraction containing attenuated recombinant LTR(7)K (having a toxic activity of about 1/260000 that of the natural one; the same as in Example 6) that had been dissolved in PBS and sterilized by filtration. 20 µl of the solution contained rotavirus vaccine, of which amount corresponded to 3.5 µg of virus particles, as well as 10 µg attenuated recombinant LTR(7)K. A preservative (0.01% thimerosal) as well as a stabilizer (0.2% porcine gelatin) was added to the solution. The solution was dispensed into appropriate containers, and was used as the oral inoculum or nasal drop of rotavirus vaccine-attenuated LT toxin. The preparation was stored at a temperature of less than 10°C in a cool and dark place.

The rotavirus vaccine prepared above was inoculated twice at a 3-week interval into mice. Then the antibody titer in the blood was evaluated. The test result showed that the ELISA antibody titer of the intranasal inoculation was 0.072 for the vaccine alone and was 0.40 for attenuated LT toxin-containing vaccine. The titer of oral inoculation was 0.020 for adjuvant-free vaccine and was 0.19 for attenuated LT toxin-containing vaccine.

### Industrial Applicability:

Examples described above demonstrate the following:
1. The adjuvant of the invention, comprising attenuated toxin, enhances the production of antibody against coexisting influenza vaccine antigen or the like.
2. Local antibody production, as well as the antibody production in the blood, is enhanced by the adjuvant of the invention when intranasally administered together with vaccine antigen.
3. The adjuvant of the invention exhibits a comparable level of immuno-potentiating activity to that of the natural one at the same dosage, even when the residual toxic activity has been reduced to an undetectable level.
4. The antibody production can be enhanced synergistically in some cases when the adjuvant of the invention is used together with another conventional adjuvant. In other words, the dose of vaccine antigen can be reduced to decrease the incidence of side reaction by using the method in which the inventive adjuvant is used together with another appropriate adjuvant.

Thus, vaccine preparations containing attenuated toxin in accordance with the present invention are useful as adjuvants with high safety. Further, vaccines using the adjuvant of the invention are not only highly safe but also are excellent vaccines having sufficient activities of enhancing immunity even when used by non-injection vaccination route, such as intranasal, percutaneous or oral ones, in which sufficient immunity is not usually expectable with any conventional methods. In addition, as seen in the Examples, the vaccines in accordance with the present invention are excellent in inducing local immunity, cellular immunity, and such which were problems difficult to overcome with publicly known adjuvants. Moreover, it is also possible to utilize natural toxins as safe adjuvants according to the present invention. Thus, because of the simplicity, the invention is easily applicable to the vaccine as compared with approaches of generating mutant toxins.

## Claims

1. An adjuvant comprising a formalin-attenuated toxin having
(i) a residual toxic activity of less than one-two thousandth (<1/2000) that of the natural toxin corresponding thereto, and
(ii) an activity of enhancing production of an antibody against influenza vaccine antigen, and
wherein said attenuated toxin has retained serine residues, glutamic acid residues, and lysine residues contained in the amino acid sequence of the natural toxin, wherein said toxin is selected from the group consisting of cholera toxin and heat-labile toxin of pathogenic *E*. *coli,* and wherein the residual toxic activity is determined by the Y-1 cell morphological transformation test.

2. The adjuvant of claim 1, wherein said toxin is a mutant having an amino acid sequence of the corresponding natural toxin wherein one or more amino acid residues are substituted, inserted, deleted, and/or added, and having an adjuvant activity.

3. The adjuvant of claim 1 or 2, wherein said toxin is a cholera toxin having a residual toxic activity of less than one-two thousandth (1/2,000) that of said corresponding natural toxin.

4. The adjuvant of claim 3, wherein said residual toxic activity is less than one-ten thousandth (1/10,000) that of said corresponding natural toxin.

5. A vaccine preparation comprising the adjuvant of any one of claims 1 to 4 and one or more vaccine antigens.

6. The vaccine preparation of claim 5, wherein said vaccine preparation is formulated for intranasal administration.

7. The vaccine preparation of claim 5, wherein said vaccine preparation is formulated for oral administration.

8. The vaccine preparation of claim 5, wherein the vaccine preparation is formulated for

9. The vaccine preparation of claim 5, wherein said vaccine comprises one or more antigens from one or more pathogenic microorganisms, said microorganisms selected from the group consisting of influenza virus, rotavirus, measles virus, rubella virus, mumps virus, AIDS virus, *Bordetella pertussis,* diphtheria bacillus, *Helicobacter pylori,* enterohaemorrhagic *Escherichia coli* (EHEC), *Chlamydia*, *Mycoplasma,* Malaria parasite, coccidium protozoa, and schistosome.

10. An adjuvant of any one of claims 1 to 4 comprising cholera toxin, further comprising an E.coli heat-labile toxin B subunit.

## Patentansprüche

1. Ein Adjuvans umfassend ein Formalin-abgemildertes Toxin mit
(i) einer verbleibenden toxischen Aktivität von weniger als ein Zweitausendstel (<1 / 2 000) im Vergleich zu dem natürlichen Toxin, das damit korrespondiert, und
(ii) einer Aktivität des Verstärkens der Produktion eines Antikörpers gegen Influenza-Vakzin-Antigen, und
wobei besagtes abgemildertes Toxin Serinreste, Glutaminsäurereste und Lysinreste zurückbehalten hat, die in der Aminosäuresequenz des natürlichen Toxins enthalten sind, wobei besagtes Toxin ausgewählt ist aus der Gruppe bestehend aus Cholera-Toxin und dem hitzelabilen Toxin von pathogenem *E*. *coli,* und wobei die verbleibende toxische Aktivität bestimmt wird durch einen Y-1-Zell-Morphologie-Transformationstest.

2. Das Adjuvans gemäß Anspruch 1, wobei besagtes Toxin eine Mutante ist mit einer Aminosäuresequenz des korrespondierenden natürlichen Toxins, wobei ein oder mehrere Aminosäurereste substituiert, insertiert, deletiert und/oder addiert sind und die Mutante eine Adjuvans-Aktivität aufweist.

3. Das Adjuvans gemäß Anspruch 1 oder 2, wobei besagtes Toxin ein Cholera-Toxin ist mit einer verbleibenden toxischen Aktivität von weniger als ein Zweitausendstel (1/2.000) im Vergleich zu derjenigen, die mit natürlichen Toxinen korrespondiert.

4. Das Adjuvans gemäß Anspruch 3, wobei die verbleibende toxische Aktivität weniger als ein Zehntausendstel (1/10.000) ist im Vergleich zu derjenigen, die mit natürlichen Toxinen korrespondiert.

5. Eine Vakzin-Herstellung, umfassend das Adjuvanz gemäß irgendeinem der Ansprüche 1 bis 4 und ein oder mehrere Vakzin-Antigene.

6. Die Vakzin-Präparation gemäß Anspruch 5, wobei besagte Vakzin-Präparation formuliert ist zur intranasalen Verabreichung.

7. Die Vakzin-Präparation gemäß Anspruch 5, wobei besagte Vakzin-Präparation formuliert ist zur oralen Verabreichung.

8. Die Vakzin-Präparation gemäß Anspruch 5, wobei besagte Vakzin-Präparation formuliert ist zur perkutanen Verabreichung.

9. Die Vakzin-Präparation gemäß Anspruch 5, wobei besagtes Vakzin ein oder mehrere Antigene umfasst von einem oder mehreren pathogenen Mikroorganismen, wobei besagte Mikroorganismen ausgewählt sind aus der Gruppe bestehend aus Influenza-Viren, Rota-Virus, Masern-Virus, Röteln-Virus, Mumps-Virus, Aids-Virus, *Bordetella pertussis,* Diphtheria-Bazillus, *Heliobacter pylori,* enterohaemorrhagischer *Escherichia coli* (EHEC), *Chlamydia, Mycoplasma,* Malaria-Parasit, Coccidium-Protozoen und Schistosoma.

10. Ein Adjuvans gemäß irgendeinem der Ansprüche 1 bis 4, umfassend Cholera-Toxin und weiter umfassend eine hitzelabile *E*. *coli*-Toxin B-Untereinheit.

## Revendications

1. Adjuvant comprenant une toxine atténuée par la formaline ayant
(i) une activité toxique résiduelle de moins de un deux millième (< 1/2 000) par rapport à celle de la toxine naturelle correspondant à celle-ci, et
(ii) une activité d'amélioration de la production d'un anticorps contre un antigène du vaccin antigrippal, et
dans lequel ladite toxine atténuée a conservé résidus de sérine, résidus d'acide glutamique et résidus de lysine contenus dans la séquence d'acides aminés de la toxine naturelle, dans lequel ladite toxine est choisie dans le groupe constitué de la toxine cholérique et la toxine thermolabile du *E.coli* pathogène, et dans lequel l'activité toxique résiduelle est déterminée par le test de transformation morphologique des cellules Y-1.

2. Adjuvant selon la revendication 1, dans lequel ladite toxine est un mutant ayant une séquence d'acides aminés de la toxine naturelle correspondante dans laquelle un ou plusieurs résidus d'acides aminés sont substitués, insérés, supprimés et/ou ajoutés, et ayant une activité d'adjuvant.

3. Adjuvant selon la revendication 1 ou 2, dans lequel ladite toxine est une toxine cholérique ayant une activité toxique résiduelle de moins de un deux millième (1/2 000) par rapport à celle de ladite toxine naturelle correspondante.

4. Adjuvant selon la revendication 3, dans lequel ladite activité toxique résiduelle est de moins de un dix millième (1/10 000) par rapport à celle de ladite toxine naturelle correspondante.

5. Préparation vaccinale comprenant l'adjuvant selon l'une quelconque des revendications 1 à 4 et un ou plusieurs antigène(s) vaccinal(aux).

6. Préparation vaccinale selon la revendication 5, dans laquelle ladite préparation vaccinale est formulée pour une administration intranasale.

7. Préparation vaccinale selon la revendication 5, dans laquelle ladite préparation vaccinale est formulée pour une administration orale.

8. Préparation vaccinale selon la revendication 5, dans laquelle la préparation vaccinale est formulée pour une administration percutanée.

9. Préparation vaccinale selon la revendication 5, dans laquelle ledit vaccin comprend un ou plusieurs antigène(s) d'un ou plusieurs micro-organisme(s) pathogène(s), lesdits micro-organismes étant choisis dans le groupe constitué du virus grippal, du rotavirus, du virus de la rougeole, du virus de la rubéole, du virus des oreillons, du virus du SIDA, du *Bordetella pertussis,* du bacille diphtérique, du *Helicobacter pylori,* du *Escherichia coli* entérohémorragique (EHEC), du *Chlamydia,* du *Mycoplasme,* du parasite de la malaria, du protozoaire coccidium et du schistosome.

10. Adjuvant selon l'une quelconque des revendications 1 à 4 comprenant la toxine cholérique, comprenant en outre une sous-unité de la toxine thermolabile B de *E. coli.*
